# EUROPEAN PATENT SPECIFICATION

(11) **EP 4 308 223 B1**
(45) Date of publication and mention of the grant of the patent: **22.04.2026**
(21) Application number: 22715587.6
(22) Date of filing: 15.03.2022
(51) Int. Cl.: A61N 5/10

(54) **SYSTEM AND METHOD FOR ASSESSING THE PERFORMANCE OF A RADIOTHERAPY APPARATUS**
SYSTEM UND VERFAHREN ZUR BEURTEILUNG DER LEISTUNG EINER STRAHLENTHERAPIEVORRICHTUNG
SYSTÈME ET PROCÉDÉ D'ÉVALUATION DE LA PERFORMANCE D'UN APPAREIL DE RADIOTHÉRAPIE

(30) Priority: 15.03.2021 GB 202103566
(43) Date of publication of application: 24.01.2024
(73) Proprietor: Elekta Limited, Crawley, West Sussex RH10 9BL (GB)
(72) Inventor: ALLEN, John, Crawley Sussex RH10 9RR (GB)
(74) Representative: Collins, Emily Jane
(86) International application number: PCT/EP2022/056746
(87) International publication number: WO 2022/194890

(56) References cited:
- WO-A1-2018/161125
- WO-A1-2018/217763
- US-A1- 2012 273 666
- KILBY W ET AL: "The CyberKnife Robotic Radiosurgery System in 2010", TECHNOLOGY IN CANCER RESEARCH & TREATMENT,, vol. 9, no. 5, 1 October 2010 (2010-10-01), pages 433 - 452, XP009158253, ISSN: 1533-0338, DOI: 10.1177/153303461000900502
- P G SEILER ET AL: "A novel tracking technique for the continuous precise measurement of tumour positions in conformal radiotherapy", PHYSICS IN MEDICINE AND BIOLOGY, 1 September 2000 (2000-09-01), ENGLAND, pages N103 - 915511910, XP055185342, Retrieved from the Internet <URL:http://iopscience.iop.org/0031-9155/45/9/402> DOI: 10.1088/0031-9155/45/9/402

## Description

This disclosure relates to radiotherapy systems and associated methods and in particular to a method of quality assurance of radiotherapy devices having tracking capabilities.

### Background

Radiotherapy can be described as the use of ionising radiation, such as X-rays, to treat a human or animal body. Radiotherapy is commonly used to treat tumours within the body of a patient or subject. In such treatments, ionising radiation is used to irradiate, and thus destroy or damage, cells which form part of the tumour.

A radiotherapy device typically comprises a gantry which supports a beam generation system, or other source of radiation, which is rotatable around a patient. For example, for a linear accelerator (linac) device, the beam generation system may comprise a source of radio frequency energy, a source of electrons, an accelerating waveguide, beam shaping apparatus, etc. Some radiotherapy devices include tracking capabilities which allow the beam to account for movement of an area which is being targeted for irradiation.

The radiotherapy apparatus is used in the delivery of therapeutic radiation to a target area, or target region, of a patient. In the case of a tumour being targeted, the radiation will damage the tumour cells beyond repair. Treatment can be delivered according to a radiotherapy treatment plan, which specifies the parameters of the treatment required by the patient, and may include information such as beam angles, beam shapes, dose-histogram-volume information and the like. The accurate delivery of treatment is crucial, as any inaccuracy in delivery will lead to radiation being deposited outside the target region, affecting healthy tissue. Thus, any movement of the target region during treatment affects the accuracy of treatment delivery, and has the potential to damage healthy tissue or sensitive organs. It is possible to appropriately restrain or sedate patients in order to restrict motion of the target region, but this will not resolve motion of internal organs (e.g. cardiac cycles, respiratory cycles).

Tracking beam delivery devices use information associated with motion of the target region to track the target region during treatment, ensuring that the correct dose is delivered to the target region. The tracking delivery compensates for target region motion such that the radiation beam is delivered to the target region throughout its motion. An accurate tracking beam delivery ensures that the dose prescribed by the treatment plan is delivered to the target region and the radiation delivered to healthy tissue is minimised.

In radiotherapy, there is a need to provide quality assurance (QA) of treatment methods for the safe delivery of treatment to the patient. A phantom, which is a specially designed object for use in measuring radiation, is commonly used in methods of QA of radiation dose delivery by a radiotherapy apparatus. However, in the case of a device with beam tracking capability such as a tracking multi-leaf collimator (MLC), there is a need for a method of QA of the tracking device which is able to account for the motion of a target region during treatment.

Existing methods of this type, as e.g. described in publication US 2012/273666 A1, utilise a specialised phantom which is capable of replicating the target region motion. This specialised phantom includes motors for moving the phantom. Due to its components, this type of phantom is expensive and complex, and additionally is only capable of a limited range of motion.

The present invention seeks to address these and other disadvantages encountered in the prior art.

### Summary of invention

Aspects and features of the present invention are described in the accompanying claims.

A first aspect is a method for controlling a radiotherapy apparatus before a therapeutic treatment, to perform quality assurance, wherein the radiotherapy apparatus comprises: a subject support surface for supporting a patient during radiotherapy; a rotatable gantry; and a beam delivery device, supported by the gantry, comprising a tracking multi-leaf collimator, MLC, for delivering a planned radiation dose to a moving target region of a patient according to a plan; wherein the method comprises: receiving motion data; controlling movement of the subject support surface to simulate motion of the target region of the patient, based on the motion data; and controlling the beam delivery device to deliver radiation according to the plan while the subject support surface simulates motion of the target region.

The beam delivery device may comprise a beam shaper.

The method can further comprise validating operation of the radiotherapy apparatus based on comparing a detected radiation dose delivered by the beam delivery device to the planned radiation dose.

The step of controlling the subject support surface to simulate motion of the target region can comprise moving the subject support surface such that a radiation detector supported by the subject support surface simulates motion of the target region.

A radiation dose delivered by the beam delivery device may be detected using a radiation detector supported by the subject support surface.

The radiation dose may comprise a dose distribution.

The motion data can correspond to at least one component of a measured and/or predicted motion of the target region of the patient.

The motion data may be based on measurements of the target region of the patient obtained using an imaging system.

The planned radiation dose can be based on the motion data.

The method may further comprise determining control instructions for the subject support surface, based on the motion data, to control the subject support surface to simulate the motion of the target region.

The method may further comprise: receiving measurement data representing a measured motion of a patient target region; and generating the motion data representing the motion of the target region based on the measurement data.

Controlling the subject support surface can comprise controlling an actuator of the subject support surface based on the motion data.

The patient may not be present on the subject support surface when the beam delivery device delivers the radiation according to the plan.

The plan may be a treatment plan for the patient.

A second aspect provided is a radiotherapy apparatus comprising: a subject support surface for supporting a patient during radiotherapy; a rotatable gantry; a beam delivery device, supported by the gantry, comprising a tracking multi-leaf collimator, MLC, for delivering a planned radiation dose to a moving target region according to a plan; and a controller configured to perform any of the methods disclosed herein.

According to another aspect, the present invention provides a computer-readable medium that stores instructions which, when executed by one or more processors of a computer device of a radiotherapy apparatus, cause the computer device to perform any of the methods disclosed herein.

### Figures

Specific embodiments are now described, by way of example only, with reference to the drawings, in which:
Figure 1 is a schematic cross sectional view of the radiotherapy apparatus;
Figure 2 is a schematic cross sectional view of a subject support surface and actuator;
Figure 3 is a flowchart setting out a method for assessing the performance of the radiotherapy apparatus;
Figure 4 is a flowchart setting out a method used in calculating table control instructions; and
Figure 5 depicts a block diagram of a computing device.

### Detailed Description

### Overview

Aspects of the disclosure will be described below. In overview, and without limitation, the present application relates to a radiotherapy apparatus comprising devices with tracking capabilities, such as a tracking MLC. In order to allow the performance of the radiotherapy apparatus to be evaluated, e.g. as part of QA, the radiotherapy apparatus is configured to move a table (referred to as a couch or subject support surface) to simulate motion of a target region of a patient. The table may be moved, based on movement data, such that a phantom placed on the couch replicates the movement of the target region - for example movement in a patient's lung. The radiotherapy apparatus may deliver a planned radiation dose for a patient target region according to a plan (e.g. a treatment plan), as the table provides simulated motion of the target region. The radiotherapy apparatus may perform the same operations to deliver a planned dose of radiation to a patient target region as if the patient was present (although the patient is not present during QA). In this way, the operation of the radiotherapy apparatus can be verified before the patient is treated. This means that a simple, static phantom can be used to test the performance of the tracking treatment system, e.g. testing the accuracy of the beam tracking, and measuring total dose delivered to the target area.

The present application may provide a more cost-effective method of performing quality assurance on a beam delivery device than the use of phantoms which use in-built motors for movement . The control system of the present application may instruct the subject support surface to move in such a manner as to cause a phantom or other QA device upon it to replicate the motion of the target region (e.g. a simulated target region).

### Radiotherapy apparatus

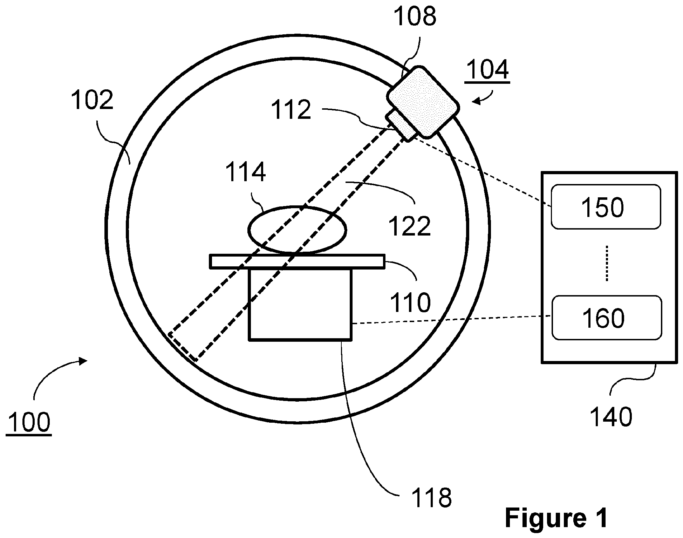

Figure 1 is a schematic cross sectional view of a radiotherapy apparatus (or radiotherapy system) 100 which comprises a beam delivery device 104. The beam delivery device 104 comprises radiation source 108 which emits a beam of therapeutic radiation 122, and a beam tracking apparatus 112 for controlling the radiation beam 122 in order for the beam 122 to track movement of a target region. The beam tracking apparatus 112 may be a beam shaper such as a diaphragm or collimator, configured to shape the beam (or in other cases a beam shaping apparatus may be separately provided). The beam tracking apparatus 112 may, for example, include a tracking multi-leaf collimator (MLC). The beam delivery device 104 can control the therapeutic radiation beam 122 to deliver a planned radiation dose to a target region of a patient according to a plan (e.g. a treatment plan).

The beam tracking apparatus 112 allows the radiotherapy apparatus 100 to account for movement of the target region during treatment. For example, the beam tracking apparatus 112 may comprise a tracking MLC which is configured to alter the positioning of its leaves in order to compensate for tumour motion.

The radiation source 108 may comprise a beam generation system. For a linac, the beam generation system may comprise a source of RF energy, an electron gun, and a waveguide.

The beam delivery device 104 is supported by a rotatable gantry 102 so as to rotate with the gantry 102. Positioned generally along a central rotational axis of the gantry 102 is a subject support surface 110 upon which, in therapeutic use, a patient 114 lies for radiotherapy treatment. The subject support surface 110 includes one or more actuators 118 which actuate the subject support surface 110 in one or more dimensions. The subject support surface 110 may be referred to as a couch, a patient support surface or a table.

The gantry 102 is configured to rotate the beam delivery device 104 around the central axis of the gantry so that the radiation beam 122 can be applied to a patient or phantom present on the subject support surface 110 from different angles.

The radiotherapy apparatus 100 further comprises a control system 140 which is connected to, and configured to control, the subject support surface 110, the beam delivery device 104 and the gantry 102. The control system 140 can comprise separate modules which may perform functions or operations such as treatment planning, treatment execution, motion tracking, motion management, and/or other tasks involved in a radiotherapy process. For example, the control system 140 comprises a beam controller 150, which is configured to control the beam delivery device to deliver a planned radiation dose to a moving target region of a patient.

The control system 140 further comprises a table controller 160 which is preferably separate in function to any control modules associated with treatment planning. The table controller 160 is configured to control the subject support surface 110, via the actuators 118, in order to move the subject support surface 110 in such a manner as to simulate motion of a target region, by performing the same movements as the target region.

The table controller 160 is separate in function to other control modules involved in delivering the planned radiation dose, in order to perform QA on the output of those control modules. The table controller module is invoked only during quality assessment, and is configured independently from the other modules of the control system. In this way, the performance of the radiotherapy apparatus 100 in delivering a planned radiation dose, e.g. based on a treatment plan, to a target region in motion can be independently assessed.

The control system 140 may be used to process input data from prior imaging, or any suitable form of information on target motion. The table controller 160 can then determine instructions for the actuator 118 to move the subject support surface 110 such that it remains stationary relative to the target region trajectory. This may involve both software and hardware components.

### Subject support surface and actuator

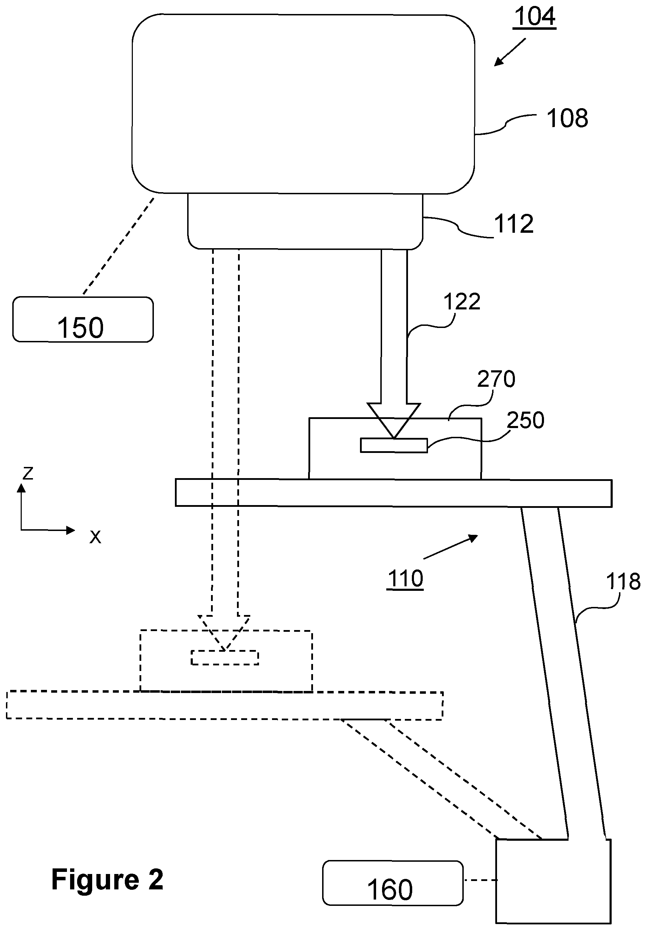

Figure 2 is a schematic cross sectional view of the subject support surface 110 and beam delivery device 104 in use.

Figure 2 shows a phantom 270 placed on the subject support surface 110. A radiation detector 250 is disposed within the phantom 270, allowing measurement of a dose distribution received at the phantom, in order to assess the performance of the radiotherapy apparatus in a QA procedure.

During beam delivery, the beam controller 150 is used to calculate and implement the required adjustments in the positioning of the beam 122 to compensate for target region motion, according to a specified treatment plan. The table controller 160 instructs the actuator 118 to move the subject support surface 110 to simulate motion of the target region.

Figure 2 shows, in solid lines, a radiation beam 122 emitted by the beam delivery device 104 at a time T1, and first position of the subject support surface 110 at time T1. The position of the radiation beam 122 is based on beam control instructions, for example in a treatment plan, which define a radiation dose to be delivered to a target region of a patient. The subject support surface is moved to simulate motion of the target region, so at time T1 the subject support surface is positioned such that the phantom is located where the target region would be. If both the beam delivery device 104 and the subject support surface are operating correctly, the radiation beam 122 is incident on the detector of the phantom at time T1, as shown in Figure 2.

Figure 2 also shows, in dashed lines, the radiation beam 122 and the subject support surface 110 at a second position at time T2. The position of the radiation beam 122 has moved in accordance with the beam control instructions, in order for the planned radiation dose to be delivered to the moving target region of the patient. As subject support surface is moved to simulate motion of the target region, at time T2 the subject support surface is positioned such that the phantom is located where the target region would be at time T2. If both the beam delivery device 104 and the subject support surface are operating correctly, the radiation beam 122 is also incident on the detector of the phantom at time T2, as shown in Figure 2.

It can be seen that the subject support surface moves in both the x and z directions in order to replicate the target region trajectory. It will be appreciated that the subject support surface may move in at least one dimension, for example, it may move in the directions shown in the figure, along the y-axis or along any combination of axes and directions. The subject support surface may also rotate in order to simulate the target region motion.

The radiation from the beam 122 is detected by the detector 250 during beam delivery, including when the target region is in motion (the motion may not be continuous). The detector 250 can then measure a number of parameters relating to treatment delivery, including, but not limited to, dose deposition and geometric accuracy of the beam. The measured values form the output of the detector 250, which may be used to assess the performance of the radiotherapy apparatus 100.

### Method of Quality Assurance

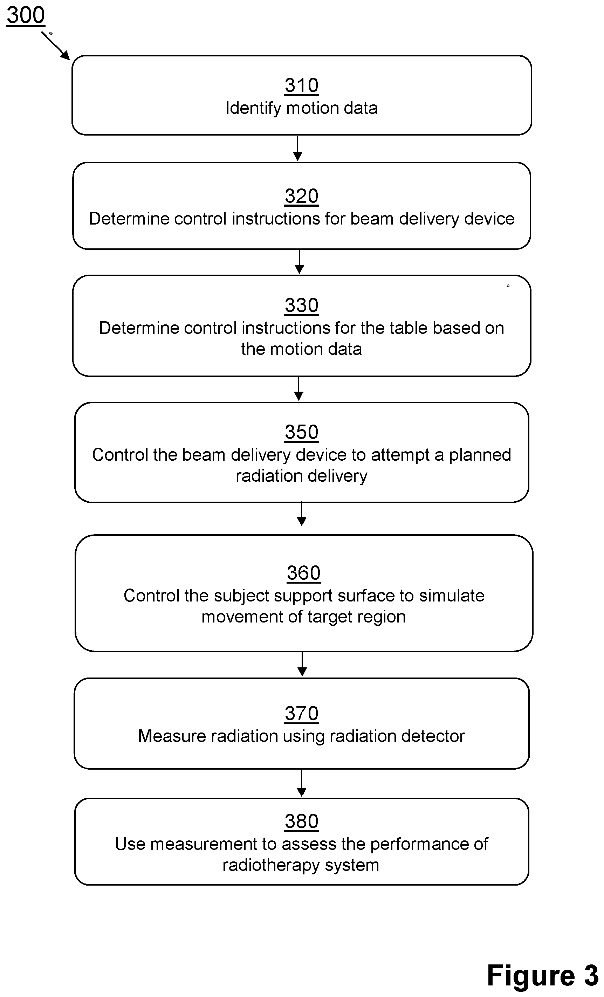

Figure 3 is a flowchart setting out a method 300 for assessing the performance of the radiotherapy apparatus 100, for example in a QA procedure. Some or all of these steps may be performed by modules of the control system 140.

In step 310, motion data is identified. The motion data may represent internal organ motion of a patient, for example the translation of a region of interest during a respiratory cycle or a cardiac cycle. The motion data may be generated based on measurements of a patient target region, for example by following a point of interest on an organ over a period of time in an imaging procedure (e.g. 4D CT). Motion data may be patient-specific.

In order to generate the motion data, information on the trajectory of the target region over a period of time may be acquired from measurement data. The measurement data may be received from an external remote system, or from an internal component of the radiotherapy apparatus (e.g. the control system 140) which has itself generated the measurement data. For example, this measurement data may be acquired from prior imaging (e.g. a CT, MRI scan) or may be acquired on-line during treatment (e.g. an MRI-linac). In an example, the motion data is derived from 4D CT data, such as a 4D CT video. The measurement data may represent internal organ motion, for example the displacement of a region of interest during a respiratory cycle or a cardiac cycle. This may be achieved by following a point of interest on the organ over a period of time. In the case of a respiratory cycle, the position of a point of interest on the lung may be identified at various time intervals whilst a subject is breathing. In this embodiment, the measurement data is used to generate motion data. Information on the trajectory of the target region over a period of time may be calculated from the measurement data representing a measured motion of a target region. Generating the resulting motion data may involve using discrete measurement data to form a continuous pattern of motion. The motion data may contain information on motion in at least one spatial dimension, and may correspond to at least one component of a measured and/or predicted motion of a target region.

In some implementations, modules of the control system may be configured to store different predefined sets of motion data representing different types of target region motion - such as respiratory motion, cardiac motion, etc. These sets may or may not be based on real measurements. The motion data may contain information on motion in at least one spatial dimension, and may correspond to at least one component of a motion of a target region. The motion data (including the one or more sets of motion data) is stored in a memory, for example in the control system 140 or in a separate memory which is operably connected to the control system 140.

Motion data defining motion of the target region can be predetermined, for example for a simulated target region. In a simple example, an amplitude of motion can be determined - for example 1cm - and a frequency can be determined - for example 0.3 Hz corresponding to 18 breaths per minute. The direction of the motion can be determined in a coordinate system of the subject support surface - for example motion may be in the x direction. Values for the amplitude, frequency, direction etc. in the motion data can be chosen based on measured values. For example, frequency can be determined based on patient breathing measurements using a spirometer.

In step 320, beam control instructions for the beam delivery device are determined using the motion data. The beam control instructions instruct the beam delivery device to perform the required adjustments in the positioning of the beam 122 to compensate for target region motion. This allows the beam delivery device to deliver a planned radiation dose to a moving target region of a patient. The beam controller 150 or another module of the control system associated with treatment planning may be used to determine the beam control instructions. This may involve identifying a trajectory of the target region from the motion data, and thus determining the corresponding adjustments of the beam delivery device 104 needed to allow the radiation beam 122 to intersect the target region during beam delivery.

The beam control instructions preferably form part of a patient's treatment plan for delivering a planned dose distribution to the target region of the patient, where the motion data has been determined based on prior imaging of the patient. Methods of generating a treatment plan, including beam tracking, are known in the art and will not be described in detail here.

In some examples, the treatment plan is a standard treatment plan not intended for patient-specific treatment, and may therefore be referred to simply as a plan. This is can be useful for QA procedures to test the correct working of the radiotherapy apparatus.

In step 330, the table control instructions are determined. The table control instructions instruct the actuator of the subject support surface 110 to perform the required movements of the subject support surface 110 to simulate the motion of the target region of the patient.

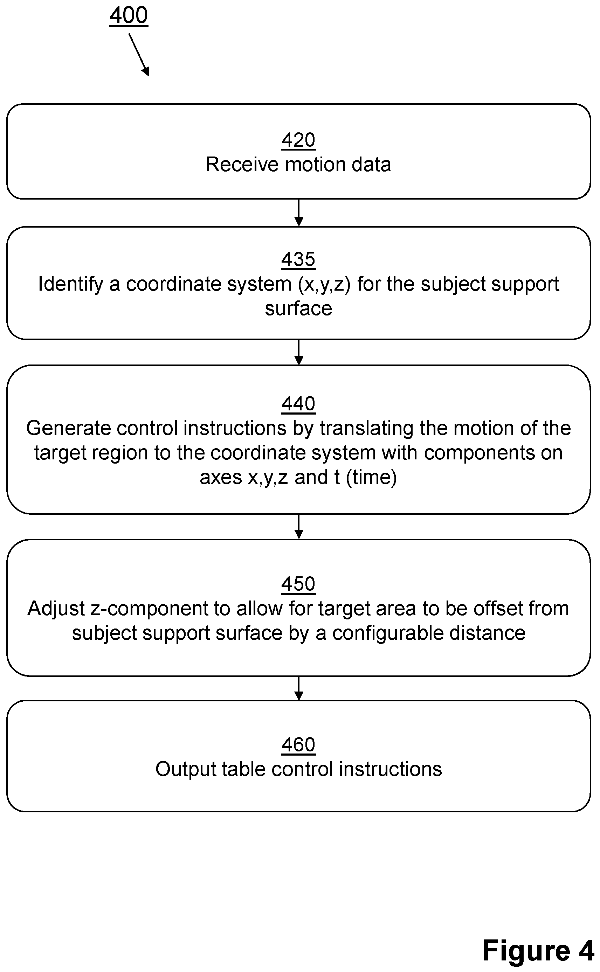

The table controller 160 may determine the table control instructions. This may involve translating the motion of the target region to a coordinate system of the subject support surface 110. This is explained further below in relation to Figure 4. These table control instructions are preferably separate from the treatment plan.

In step 350, the beam delivery device 104 is controlled to deliver (or attempt to deliver) a planned radiation dose, based on the beam control instructions (e.g. as part of the patient treatment plan). The beam delivery device performs the same operations to deliver a planned dose of radiation to a patient target region as if the patient was present. In this way, the operation of the beam delivery device 104 and radiotherapy apparatus as a whole can be verified before the patient is present.

In some implementations, the beam delivery device includes an MLC as the beam tracking apparatus, and the leaves of the MLC are controlled to move the beam 122 in order to track the target region.

The required adjustments in the positioning of the beam 122 in order for it to intersect the target region while the target region is in motion are implemented by the beam tracking apparatus. This may involve continuous adjustments to the positioning of the beam 122, or adjustments to the positioning of the beam 122 at discrete intervals.

The beam delivery device 104 may be controlled based on the beam control instructions and further based on a phase control signal, for example a measurement of current breathing of a patient. The phase control signal is used to ensure that the movement of the radiation beam is phase locked to real time movements of a patient target area. Optionally, the phase control signal may be a predefined waveform representing e.g. respiratory motion.

In step 360, the subject support surface 110 is moved, based on the table control instructions, such that it simulates the target region motion. This is done simultaneously as the beam delivery device 104 delivers radiation.

The subject support surface 110 moves such that a phantom (and/or radiation detector) resting on the subject support surface 110 replicates the motion of the target region, e.g. follows the trajectory of the simulated target region. Thus, the target motion is replicated by the subject support surface 110, and the radiation beam 122 is delivered to a static phantom including a radiation detector, resting on the subject support surface according to the treatment plan specified for the target region in motion.

The subject support surface 110 may be moved based on the table control instructions and additionally subject support surface based on a phase control signal used to ensure that the movement of the subject support surface is phase locked to real time movements of a patient target area, and/or phase locked to the movement of the radiation beam. The phase control signal may be the same as the phase control signal used to control the beam delivery device 104 - for example where the phase control signal is a measurement of current breathing of a patient. The phase control signal may be provided by a spirometer.

At step 370, radiation delivered to the target region by the beam of radiation is measured using a radiation detector (also referred to as a radiation measurement device) positioned on the subject support surface. The radiation detector is disposed within a phantom which in turn is supported by the subject support surface 110, as shown in Figure 2.

The radiation detector is configured to measure the radiation delivered to the target region throughout the beam delivery, in order to measure the total dose delivered. Specifically, the radiation detector may measure a dose distribution. This can be compared to a planned dose distribution to be delivered to the moving target area, for example as defined in the patient treatment plan.

The radiation detector may output one or more measurements to one or more modules of the control system 140. For example, the output can include, but is not limited to, the dose deposited at the target region, and the geometric accuracy of the beam delivery.

If the radiation detector is radiographic film, the film is retrieved (e.g. from within the phantom) and assessed either visually or by an image processing system.

Radiation deposited in the target region can be determined by measuring the radiation incident on the detector during beam delivery, and the resulting measurement may be used to compute the dose deposited in the target region. The measurement of the geometric accuracy of the beam may vary depending on the radiation detector, and may be obtained in the appropriate manner depending on the device used. For example, if a radiographic film were used, the geometric accuracy of the beam could be determined by the sharpness of the resulting shadow on the film. A sharp shadow would indicate a more accurate beam delivery, and a blurred shadow would indicate an inaccurate beam delivery.

At step 380, the measurement value or values output by the radiation detector are used to assess the performance of the radiotherapy apparatus. This may include determining whether the dose specified in the treatment plan has been delivered to the target region (within an allowable margin of error), and/or whether the geometric accuracy of the beam was within an allowable margin of error. This indicates if both the beam delivery device 104 and the subject support surface are operating correctly. The performance of the radiotherapy apparatus 100 is then assessed based on whether these predetermined criteria of the treatment plan are satisfied. If the predetermined criteria are satisfied, then this is treated as a validation of operation of the radiotherapy apparatus.

Validation of operation of the radiotherapy apparatus may be performed automatically by the control system 140, or may be carried out by a human specialist.

A module of the control system 140, or another computing device, may be used to compare the output of the detector with the predetermined treatment plan in order to assess whether the predetermined criteria have been satisfied. This may include computing an allowable margin of error on each value of the predetermined criteria, and determining whether each respective measured value of the output falls within the appropriate margin of error on the treatment plan. The module of the control system, or computing device, can then use this information to assess the performance of the radiotherapy apparatus.

Figure 4 is a flowchart setting out a method 400 used to calculate table control instructions.

In step 420, data representing the motion of the target region is received - for example received from an imaging system or retrieved from a memory of a module of the control system 140. The motion data may contain information on motion in at least one spatial dimension, and may correspond to at least one component of a measured and/or predicted motion of a target region in a period of time.

In step 435, a coordinate system for the subject support surface 110 is identified by a modules of the control system 140. This may be a cartesian (x,y,z) coordinate system, or any other suitable coordinate system. The coordinate system may be allocated to the subject support surface 110, or may have been previously determined for the subject support surface 110.

In step 440, the table controller 160 generates the control instructions by translating the identified motion of the target region to the coordinate system identified for the subject support surface 110, including a time component. In cases where the measurement data has been acquired in an equivalent coordinate system, this may involve translating the coordinates of the identified motion of the target region such that the motion remains within the range of motion of the subject support surface 110. In cases where the measurement data has not been acquired in an equivalent coordinate system, a suitable algorithm or any other appropriate means may be applied to translate the identified motion into the coordinate system of the subject support surface 110.

In step 450, one or more of the components can be adjusted to allow for the target region trajectory to be offset from the subject support surface by a configurable distance. This allows the target region trajectory to be aligned with, for example, a radiation detector resting on the subject support surface 110.

In step 460, the resulting table control instructions are output, e.g. to the actuator 118.

### Modifications and alternatives

Any implementations, examples, embodiments and the like described herein can be combined.

It will be understood that the description of specific implementations, examples, embodiments and the like is by way of example only and is not intended to limit the scope of the present disclosure. Many modifications of the described embodiments, some of which are now described, are envisaged and intended to be within the scope of the present disclosure.

### Radiotherapy apparatus

The beam delivery device 104 may comprise a heavy metal target towards which high energy electrons are directed. When the electrons strike the target, X-rays are produced in a variety of directions. A primary collimator (not shown) may block X-rays travelling in certain directions and pass only forward travelling X-rays to produce a treatment beam. The X-rays may be filtered and may pass through one or more ion chambers for dose measuring. In some implementations, the source of radiation is configured to emit either an X-ray beam or a particle beam. Such implementations allow the device to provide particle beam therapy, i.e. a type of external beam therapy where particles (e.g. protons or light ions), rather than X-rays, are directed towards the target region.

In some examples, the radiation source and the beam shaping apparatus are provided as a single unit, or alternatively the radiation source and the beam shaping unit may be provided separately.

It will be appreciated that the gantry may be replaced with one or more apparatus which allows the beam delivery device to rotate around an axis of rotation.

### Beam tracking apparatus

The beam tracking apparatus 112 may comprise a tracking MLC which is configured to alter the positioning of individual leaves in order to compensate for tumour motion. An MLC include two banks of leaves, forming two opposing arrays. Each leaf can be individually extended into and out of the path of the radiation beam 122 in order to shape the beam. The leaves of the MLC may be controlled to take different positions to selectively block some or all of the radiation beam 122, in order to alter the shape of the beam. The leaves of the MLC may be manipulated to shape the beam such that the beam continuously intersects the target region during beam delivery. This may be combined with another beam shaping apparatus, for example a diaphragm, in order to extend the beam shaping capability, and thus the tracking capability, of the beam tracking apparatus.

In some implementations, the beam tracking apparatus 112 includes a bank of motors, with each motor configured to move a corresponding one of the leaves. Movement of each leaf by the motors is controlled by the beam controller 150. For example, the beam controller 150 can control leaf movement using the motors to shape the radiation beam 122. The leaves can then be controlled to shape the beam such that the beam continuously intersects the target region during beam delivery.

### Subject support surface

The subject support surface 110 and actuators 118 can include a device which supports adjustments in the positioning of the couch, such as a 'hexapod', which can alter the position of the couch with up to six degrees of freedom (x, y, z, pitch, roll and yaw).

Any suitable coordinate system may identified for the subject support surface 110, where a suitable coordinate system can represent motion in at least one spatial dimension and/or direction.

The subject support surface (also referred to as a table or couch) can include different layers, such as cushioning, and additional elements such as patient positioning devices (e.g. leg supports) can be used with the subject support surface. Therefore, references to the subject support surface will be understood to encompass additional elements and layers such as these.

### Radiation detectors

The radiation detector placed on the subject support surface 110 may comprise any suitable means of measuring radiation. This may include, and is not limited to, at least one of a phantom, a dosimeter, a farmer chamber, a detector array, radiographic film or any other suitable device.

The phantom described herein may or may not include a radiation detector. Also, the radiation detector may be used in the described method with or without the phantom.

Additional quality assurance devices can be combined with the methods outlined above, in order to obtain additional information on treatment delivery.

### Motion data identification

The steps 320 and 330 can be determined concurrently or in any order. The motion data utilised in steps 320 and 320 may not be the same motion data. For example, motion data based on a measured motion of a target region within a patient may be used in step 320, and motion data from a predefined model may be used in step 330.

The motion data may be based on measured motion of a target region within a patient. For example, it may be measurement data received from an imaging device. However the motion data may alternatively or additionally be preloaded data which is saved in memory and accessed by the control system or a module of the control system.

Preloaded data may be input into the control system 140 for use in determining the instructions for the beam delivery device 104 and/or the actuators 118. This data may represent a motion of a target region measured at a previous point in time. This can include motions previously calculated by the table controller 160 to represent the motion of a target region, which were saved to the memory of an external hardware (e.g. a computer). This can also include the output of a measurement device, such as a spirometer, which was used to measure a patient motion at a previous point in time. A spirometer can be used to measure the breathing cycle of a patient, and the output of the spirometer may be used as an input for one or more of the modules of the control system 140, in order to account for respiratory motion.

Another embodiment may involve saving the preloaded data to the memory of one or more of the hardware components of the control system 140.

The preloaded data may have been constructed to represent a motion of a target region based on known characteristics of that motion. This could be achieved using equations or algorithms which calculate a known characteristic motion of a target region. This data may also be stored on an internal or external hardware component of the control system 140. This may otherwise involve using a predefined model of a known motion, e.g. a respiratory cycle, to represent the motion of a target region.

In another embodiment, the coordinates describing the motion of a target region may be input during beam delivery, with the table controller 160 using this data to calculate the required coordinates for the subject support surface 110 in 'real-time'. This may mimic a treatment in which real-time imaging of the motion of the target region during treatment dictates the adjustments made to the subject support surface 110. This may involve one or more fiducial markers being placed on or in the phantom 270, in order to trigger the real-time imaging. The location of the fiducial marker throughout the motion of the target region can then be used to determine the required adjustments of the subject support surface 110 to compensate for the motion.

The apparatus described may also be used to 'stress test' a treatment plan prescribed to a patient. Motion data involving atypical patient movements during treatment (e.g. coughing, unpredictable movements) can be used to determine the control instructions for the subject support surface 110. The response of the radiotherapy apparatus to such atypical movements can then be discerned during QA, which may be useful in assessing the safety of a treatment plan.

### Control system

The control system is a computing device, computer, processor, or other processing apparatus. The control system may be formed by several discrete processors. The control system can be provided by multiple separate controllers (separate in hardware and/or software), and may include distributed components. This can include physical and functional components in different elements of the system, such as in the beam tracking apparatus 112 and the actuators 118.

Software components of the control system 140 may include operation device software, application software, etc. These software components may have in-built algorithms or processes for extracting information on target motion from a suitable form of motion data, and may use further algorithms or processes to determine control instructions based on this information.

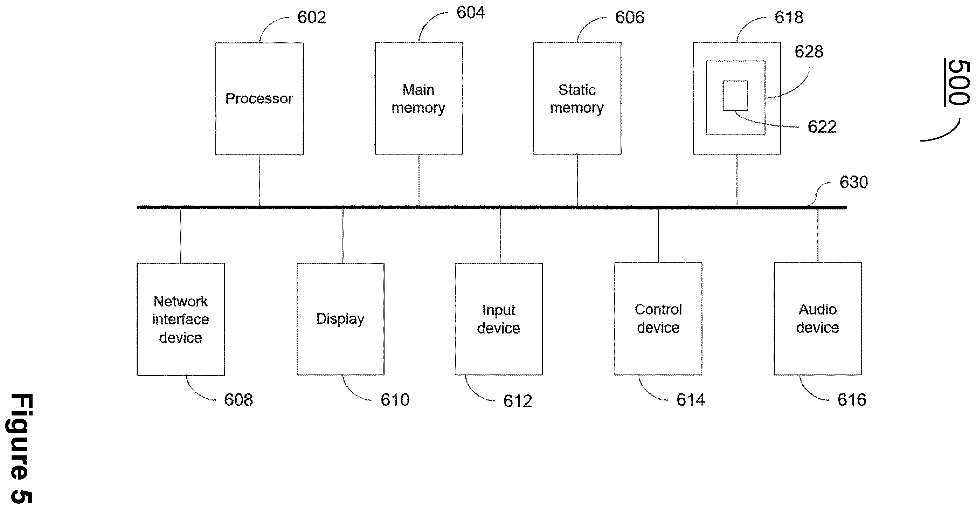

Figure 5 is a block diagram of one implementation of a computing device 500 within which a set of instructions, for causing the computing device to perform any one or more of the methodologies discussed herein, may be executed. In alternative implementations, the computing device may be connected (e.g., networked) to other machines in a Local Area Network (LAN), an intranet, an extranet, or the Internet. The computing device may operate in the capacity of a server or a client machine in a client-server network environment, or as a peer machine in a peer-to-peer (or distributed) network environment. The computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 500 includes a processing device 602, a main memory 604 (e.g., read-only memory (ROM), flash memory, dynamic random access memory (DRAM) such as synchronous DRAM (SDRAM) or Rambus DRAM (RDRAM), etc.), a static memory 606 (e.g., flash memory, static random access memory (SRAM), etc.), and a secondary memory (e.g., a data storage device 618), which communicate with each other via a bus 630.

Processing device 602 represents one or more general-purpose processors such as a microprocessor, central processing unit, or the like. More particularly, the processing device 602 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 602 may also be one or more special-purpose processing devices such as an application specific integrated circuit (ASIC), a field programmable gate array (FPGA), a digital signal processor (DSP), network processor, or the like. Processing device 602 is configured to execute the processing logic (instructions 622) for performing the operations and steps discussed herein.

The computing device 500 may further include a network interface device 608. The computing device 500 also may include a video display unit 610 (e.g., a liquid crystal display (LCD) or a cathode ray tube (CRT)), an alphanumeric input device 612 (e.g., a keyboard or touchscreen), a cursor control device 614 (e.g., a mouse or touchscreen), and an audio device 616 (e.g., a speaker).

The data storage device 618 may include one or more machine-readable storage media (or more specifically one or more non-transitory computer-readable storage media) 628 on which is stored one or more sets of instructions 622 embodying any one or more of the methodologies or functions described herein. The instructions 622 may also reside, completely or at least partially, within the main memory 604 and/or within the processing device 602 during execution thereof by the computer system 500, the main memory 604 and the processing device 602 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations. Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as " receiving", "determining", "comparing ", "enabling", "maintaining," "identifying," "applying, " "transmitting," "generating," or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

The approaches described herein may be embodied on a computer-readable medium, which may be a non-transitory computer-readable medium. The computer-readable medium may carry computer-readable instructions arranged for execution upon a processor so as to cause the processor to carry out any or all of the methods described herein.

The term "computer-readable medium" as used herein refers to any medium that stores data and/or instructions for causing a processor to operate in a specific manner. Such storage medium may comprise non-volatile media and/or volatile media. Non-volatile media may include, for example, optical or magnetic disks. Volatile media may include dynamic memory. Exemplary forms of storage medium include, a floppy disk, a flexible disk, a hard disk, a solid state drive, a magnetic tape, or any other magnetic data storage medium, a CD-ROM, any other optical data storage medium, any physical medium with one or more patterns of holes, a RAM, a PROM, an EPROM, a FLASH-EPROM, NVRAM, and any other memory chip or cartridge.

It is to be understood that the above description is intended to be illustrative, and not restrictive. Many other implementations will be apparent to those of skill in the art upon reading and understanding the above description.

The invention is defined by the appended claims.

## Claims

1. A method for controlling a radiotherapy apparatus before a therapeutic treatment to perform quality assurance.
wherein the radiotherapy apparatus comprises:
a subject support surface for supporting a patient during radiotherapy;
a rotatable gantry; and
a beam delivery device, supported by the gantry, comprising a tracking multi-leaf collimator, MLC, for delivering a planned radiation dose to a moving target region of a patient according to a plan, the plan comprising instructions for instructing the beam delivery device to adjust a positioning of a beam to compensate for motion of the target region;
wherein the method comprises:
receiving motion data;
controlling movement of the subject support surface to simulate motion of the target region of the patient, based on the motion data; and
controlling the beam delivery device to deliver radiation according to the plan while the subject support surface simulates motion of the target region.

2. The method of claim 1, wherein the beam delivery device comprises a beam shaper.

3. The method of claim 1 or 2, further comprising validating operation of the radiotherapy apparatus based on comparing a detected radiation dose delivered by the beam delivery device to the planned radiation dose.

4. The method of any preceding claim, wherein the step of controlling the subject support surface to simulate motion of the target region comprises moving the subject support surface such that a radiation detector supported by the subject support surface simulates motion of the target region.

5. The method of any preceding claim, further comprising detecting a radiation dose delivered by the beam delivery device using a radiation detector supported by the subject support surface.

6. The method of any preceding claim, wherein the planned radiation dose comprises a dose distribution.

7. The method of any preceding claim, wherein the motion data corresponds to at least one component of a measured and/or predicted motion of the target region of the patient.

8. The method of any preceding claim, wherein the motion data is based on measurements of the target region of the patient obtained using an imaging system.

9. The method of any preceding claim, wherein the planned radiation dose is based on the motion data.

10. The method of any preceding claim, further comprising determining control instructions for the subject support surface, based on the motion data, to control the subject support surface to simulate the motion of the target region.

11. The method of any preceding claim, further comprising:
receiving measurement data representing a measured motion of a patient target region; and
generating the motion data representing the motion of the target region based on the measurement data.

12. The method of any preceding claim, wherein controlling the subject support surface comprises controlling an actuator of the subject support surface based on the motion data.

13. The method of any preceding claim, wherein the plan is a treatment plan for the patient.

14. A radiotherapy apparatus comprising:
a subject support surface for supporting a patient during radiotherapy;
a rotatable gantry;
a beam delivery device, supported by the gantry, comprising a tracking multi-leaf collimator, MLC, for delivering a planned radiation dose to a moving target region according to a plan, the plan comprising instructions for instructing the beam delivery device to adjust a positioning of a beam to compensate for motion of the target region; and
a controller configured to perform a method according to any preceding claim.

15. A computer-readable medium that stores instructions which, when executed by one or more processors of a computer device of a radiotherapy apparatus of claim 14, cause the computer device to perform a method according to any of claims 1 to 13.

## Patentansprüche

1. Verfahren zum Steuern einer Strahlentherapievorrichtung vor einer therapeutischen Behandlung, um eine Qualitätssicherung durchzuführen, wobei die Strahlentherapievorrichtung umfasst:
eine Subjekt-Tragefläche zum Lagern eines Patienten während der Strahlentherapie;
eine drehbare Gantry; und
eine von der Gantry getragene Strahlabgabevorrichtung,
umfassend einen nachgeführten Mehrblattkollimator, MLC (Multi-Leaf Collimator), zur Abgabe einer geplanten Strahlendosis an eine sich bewegende Zielregion eines Patienten nach einem Plan, wobei der Plan Anweisungen umfasst, um die Strahlabgabevorrichtung anzuweisen, eine Positionierung eines Strahls anzupassen, um die Bewegung der Zielregion auszugleichen;
wobei das Verfahren Folgendes umfasst:
Empfangen von Bewegungsdaten;
Steuern der Bewegung der Subjekt-Tragefläche, um die Bewegung der Zielregion des Patienten zu simulieren, basierend auf den Bewegungsdaten; und
Steuern der Strahlabgabevorrichtung, um Strahlung entsprechend dem Plan abzugeben, während die Subjekt-Tragefläche die Bewegung der Zielregion simuliert.

2. Verfahren nach Anspruch 1, wobei die Strahlabgabevorrichtung einen Strahlformer umfasst.

3. Verfahren nach Anspruch 1 oder 2, das ferner umfasst, den Betrieb der Strahlentherapievorrichtung zu validieren, basierend auf einem Vergleich der durch die Strahlabgabevorrichtung abgegebenen Strahlendosis mit der geplanten Strahlendosis.

4. Verfahren nach einem der vorhergehenden Ansprüche, wobei der Schritt des Steuerns der Subjekt-Tragefläche, um eine Bewegung der Zielregion zu simulieren, umfasst, die Subjekt-Tragefläche so zu bewegen, dass ein von der Subjekt-Tragefläche getragener Strahlungsdetektor eine Bewegung der Zielregion simuliert.

5. Verfahren nach einem der vorhergehenden Ansprüche, das ferner umfasst, eine durch die Strahlabgabevorrichtung abgegebene Strahlendosis mit Hilfe eines von der Subjekt-Tragefläche getragenen Strahlungsdetektors zu detektieren.

6. Verfahren nach einem der vorhergehenden Ansprüche, wobei die geplante Strahlendosis eine Dosisverteilung umfasst.

7. Verfahren nach einem der vorhergehenden Ansprüche, wobei die Bewegungsdaten mindestens einer Komponente einer gemessenen und/oder vorhergesagten Bewegung der Zielregion des Patienten entsprechen.

8. Verfahren nach einem der vorhergehenden Ansprüche, wobei die Bewegungsdaten auf Messungen der Zielregion des Patienten basieren, die unter Verwendung eines bildgebenden Systems erhalten wurden.

9. Verfahren nach einem der vorhergehenden Ansprüche, wobei die geplante Strahlendosis auf den Bewegungsdaten basiert.

10. Verfahren nach einem der vorhergehenden Ansprüche, das ferner umfasst, Steueranweisungen für die Subjekt-Tragefläche zu bestimmen, basierend auf den Bewegungsdaten, um die Subjekt-Tragefläche zu steuern, um die Bewegung der Zielregion zu simulieren.

11. Verfahren nach einem der vorhergehenden Ansprüche, das ferner Folgendes umfasst:
Empfangen von Messdaten, die eine gemessene Bewegung einer Zielregion des Patienten darstellen; und
Erzeugen der Bewegungsdaten, die die Bewegung der Zielregion darstellen, basierend auf den Messdaten.

12. Verfahren nach einem der vorhergehenden Ansprüche, wobei das Steuern der Subjekt-Tragefläche umfasst, einen Aktuator der Subjekt-Tragefläche zu steuern, basierend auf den Bewegungsdaten.

13. Verfahren nach einem der vorhergehenden Ansprüche, wobei der Plan ein Behandlungsplan für den Patienten ist.

14. Strahlentherapievorrichtung, die Folgendes umfasst:
eine Subjekt-Tragefläche zum Lagern eines Patienten während der Strahlentherapie;
eine drehbare Gantry;
eine von der Gantry getragene Strahlabgabevorrichtung, umfassend einen nachgeführten Mehrblattkollimator, MLC, zur Abgabe einer geplanten Strahlendosis an eine sich bewegende Zielregion nach einem Plan, wobei der Plan Anweisungen umfasst, um die Strahlabgabevorrichtung anzuweisen, eine Positionierung eines Strahls anzupassen, um die Bewegung der Zielregion auszugleichen; und
eine Steuerung, die dazu ausgelegt ist, ein Verfahren nach einem der vorhergehenden Ansprüche durchzuführen.

15. Computerlesbares Medium, das Anweisungen speichert, die, wenn sie von einem oder mehreren Prozessoren einer Computervorrichtung einer Strahlentherapievorrichtung nach Anspruch 14 ausgeführt werden, die Computervorrichtung veranlassen, ein Verfahren nach einem der Ansprüche 1 bis 13 durchzuführen.

## Revendications

1. Procédé de commande d'un appareil de radiothérapie avant un traitement thérapeutique pour réaliser une assurance qualité,
l'appareil de radiothérapie comprenant :
une surface de support de sujet pour le patient pendant la radiothérapie ;
un portique rotatif ; et
un dispositif d'administration de faisceau, supporté par le portique, comprenant un collimateur multilame de suivi, MLC, pour administrer une dose de rayonnement planifiée à une région cible mobile d'un patient conformément à un plan, le plan comprenant des instructions pour ordonner au dispositif d'administration de faisceau d'ajuster le positionnement d'un faisceau afin de compenser le mouvement de la région cible ;
le procédé comprenant :
la réception de données de mouvement ;
la commande du mouvement de la surface de support de sujet pour simuler le mouvement de la région cible du patient, sur la base des données de mouvement ; et
la commande du dispositif d'administration de faisceau pour administrer un rayonnement conformément au plan tandis que la surface de support de sujet simule le mouvement de la région cible.

2. Procédé selon la revendication 1, le dispositif d'administration de faisceau comprenant un conformateur de faisceau.

3. Procédé selon la revendication 1 ou 2, comprenant en outre la validation du fonctionnement de l'appareil de radiothérapie sur la base de la comparaison d'une dose de rayonnement détectée administrée par le dispositif d'administration de faisceau à la dose de rayonnement planifiée.

4. Procédé selon l'une quelconque des revendications précédentes, l'étape de commande de la surface de support de sujet pour simuler le mouvement de la région cible comprenant le déplacement de la surface de support de sujet de telle sorte qu'un détecteur de rayonnement supporté par la surface de support de sujet simule le mouvement de la région cible.

5. Procédé selon l'une quelconque des revendications précédentes, comprenant en outre la détection d'une dose de rayonnement administrée par le dispositif d'administration de faisceau à l'aide d'un détecteur de rayonnement supporté par la surface de support de sujet.

6. Procédé selon l'une quelconque des revendications précédentes, la dose de rayonnement planifiée comprenant une distribution de dose.

7. Procédé selon l'une quelconque des revendications précédentes, les données de mouvement correspondant à au moins une composante d'un mouvement mesuré et/ou prédit de la région cible du patient.

8. Procédé selon l'une quelconque des revendications précédentes, les données de mouvement étant basées sur des mesures de la région cible du patient obtenues à l'aide d'un système d'imagerie.

9. Procédé selon l'une quelconque des revendications précédentes, la dose de rayonnement planifiée étant basée sur les données de mouvement.

10. Procédé selon l'une quelconque des revendications précédentes, comprenant en outre la détermination d'instructions de commande pour la surface de support de sujet, sur la base des données de mouvement, pour commander la surface de support de sujet afin de simuler le mouvement de la région cible.

11. Procédé selon l'une quelconque des revendications précédentes, comprenant en outre :
la réception de données de mesure représentant un mouvement mesuré d'une région cible du patient ; et
la génération des données de mouvement représentant le mouvement de la région cible sur la base des données de mesure.

12. Procédé selon l'une quelconque des revendications précédentes, la commande de la surface de support de sujet comprenant la commande d'un actionneur de la surface de support de sujet sur la base des données de mouvement.

13. Procédé selon l'une quelconque des revendications précédentes, le plan étant un plan de traitement pour le patient.

14. Appareil de radiothérapie comprenant :
une surface de support de sujet pour le patient pendant la radiothérapie ;
un portique rotatif ;
un dispositif d'administration de faisceau, supporté par le portique, comprenant un collimateur multilame de suivi, MLC, pour administrer une dose de rayonnement planifiée à une région cible mobile conformément à un plan, le plan comprenant des instructions pour ordonner au dispositif d'administration de faisceau d'ajuster un positionnement d'un faisceau pour compenser le mouvement de la région cible ; et
un dispositif de commande configuré pour réaliser un procédé selon l'une quelconque des revendications précédentes.

15. Support lisible par ordinateur qui stocke des instructions qui, lorsqu'elles sont exécutées par un ou plusieurs processeurs d'un dispositif informatique d'un appareil de radiothérapie selon la revendication 14, amènent le dispositif informatique à réaliser un procédé selon l'une quelconque des revendications 1 à 13.
